(19) Europäisches Patentamt
European Patent Office
Office européen des brevets

(11) Veröffentlichungsnummer: **0 313 981**
**A2**

(12) **EUROPÄISCHE PATENTANMELDUNG**

(21) Anmeldenummer: **88117451.0**

(51) Int. Cl.⁴: **C07D 249/08 , A61K 31/41**

(22) Anmeldetag: **20.10.88**

(30) Priorität: **30.10.87 DE 3736773**

(43) Veröffentlichungstag der Anmeldung:
**03.05.89 Patentblatt 89/18**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Büchel, Karl Heinz, Prof. Dr.**
**Dabringhauser Strasse 42**
**D-5093 Burscheid(DE)**
Erfinder: **Krämer, Wolfgang, Dr.**
**Rosenkranz 25**
**D-5093 Burscheid 2(DE)**
Erfinder: **Plempel, Manfred, Dr.**
**Zwengenberger Strasse 3 c**
**D-5657 Haan(DE)**
Erfinder: **Ritter, Wolfgang, Dr.**
**August-Jung-Weg 45**
**D-5600 Wuppertal 1(DE)**

(54) **3-(4-Chlorphenyl)-2,2-dimethyl-3-hydroxy-4-(1,2,4-triazol-1-yl)-butyronitril.**

(57) Die Erfindung betrifft die neue Verbindung 3-(4-Chlorphenyl)-2,2-dimethyl-3-hydroxy-4-(1,2,4-triazol-1-yl)-butyronitril, ein Verfahren zur ihrer Herstellung und ihre Verwendung bei der Bekämpfung von Krankheiten, insbesondere Mykosen.

EP 0 313 981 A2

## 3-(4-Chlorphenyl)-2,2-dimethyl-3-hydroxy-4-(1,2,4-triazol-1-yl)-butyronitril

Die Erfindung betrifft die neue Verbindung 3-(4-Chlorphenyl)-2,2-dimethyl-3-hydroxy-4-(1,2,3-triazol-1-yl)-butyronitril, ein Verfahren zur ihrer Herstellung und ihre Verwendung bei der Bekämpfung von Krankheiten, insbesondere Mykosen.

Es ist bereits bekannt, daß bestimmte Triazolylbutyronitrile, wie besipielsweise die Verbindung 3-(4-Chlorphenyl)-3-hydroxy-2-methyl-4-(1,2,4-triazol-1-yl)-butyronitril gute antimykotische Eigenschaften besitzen (vgl. EP 106 515).

Die Wirksamkeit dieser vorbekannten Verbindungen ist jedoch nicht in allen Indikationen völlig zufriedenstellend.

Es wurde die neue Verbindung 3-(4-Chlorphenyl)-2,2-dimethyl-3-hydroxy-4-(1,2,4-triazol-1-yl)-butyronitril der Formel (I),

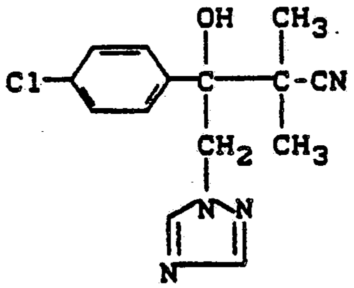

(I)

sowie deren physiologisch verträgliche Säureadditionssalze gefunden.

Die Verbindung der Formel (I) kann in Form von optischen Isomeren oder Isomerengemischen unterschiedlicher Zusammensetzung vorliegen. Sowohl die reinen Isomeren als auch die Isomerengemische werden erfindungsgemäß beansprucht.

Weiterhin wurde gefunden, daß man die neue Verbindung 3-(4-Chlorphenyl)-2,2-dimethyl-3-hydroxy-4-(1,2,4-triazol-1-yl)-butyronitril der Formel (I)

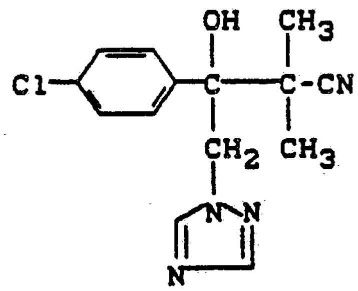

(I)

erhält, wenn man die Verbindung 3-(4-Chlorphenyl-2,2-dimethyl-3-hydroxy-4-(1,2,4-triazol-1-yl)-butyraldehydoxim der Formel (II)

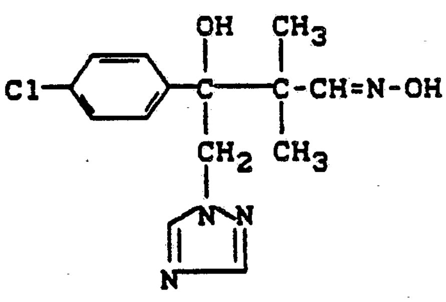

(II)

mit einem wasserabspaltenden Mittel, gegebenenfalls in Gegenwart eines Verdünnungsmittels und gegebenenfalls in Gegenwart eines Reaktionshilfsmittels umsetzt und gegebenenfalls anschließend eine Säure addiert.

Schließlich wurde gefunden, daß die neue Verbindung 3-(4-Chlorphenyl)-2,2-dimethyl-3-hydroxy-4-(1,2,4-triazol-1-yl)-butyronitril der Formel (I) gute antimikrobielle, insbesondere gute antimykotische Eigenschaften besitzt.

Überraschenderweise zeigt die neue Verbindung 3-(4-Chlorphenyl)-2,2-dimethyl-3-hydroxy-4-(1,2,4-triazol-1-yl)-butyronitril der Formel (I) in bestimmten Indikationen eine deutlich bessere antimykotische Wirksamkeit als die aus dem Stand der Technik bekannten Triazolylbutyronitrile, wie besipielsweise die Verbindung 3-(4-Chlorphenyl)-3-hydroxy-2-methyl-4-(1,2,4-triazol-1-yl)-butyronitril, welches eine chemisch und wirkungsmäßig naheliegende Verbindung ist.

Verwendet man 3-(4-Chlorphenyl)-2,2-dimethyl-3-hydroxy-4-(1,2,4-triazol-1-yl)-butyraldehydoxim als Ausgangsverbindung und Acetanhydrid als wasserabspaltendes Mit tel, so läßt sich der Reaktionsablauf des erfindungsgemäßen Verfahrens durch das folgende Formelschema darstellen:

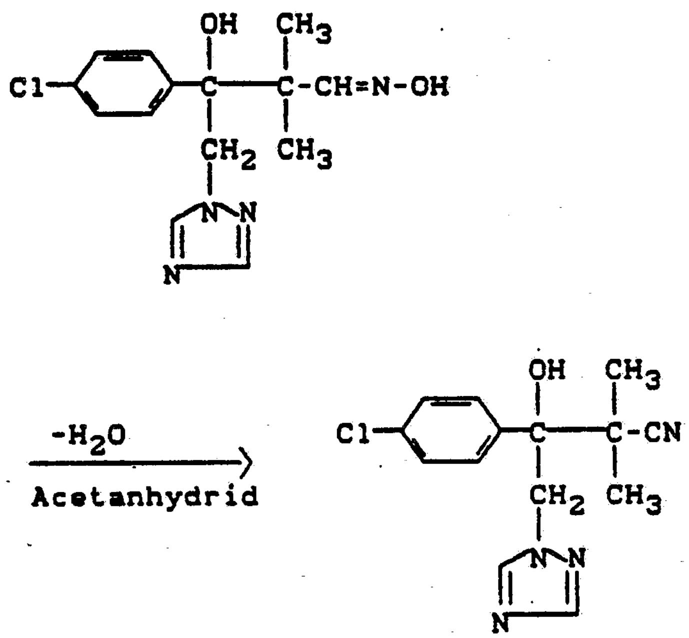


Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Die zur Durchführung des erfindungsgemäßen Verfahrens als Ausgangsstoff benötigte Verbindung 3-(4-Chlorphenyl)-2,2-dimethyl-3-hydroxy-4-(1,2,4-triazol-1-yl)butyraldehydoxim der Formel (II) ist noch nicht bekannt.

Man erhält sie, wenn man die Verbindung 2-(4-Chlorphenyl)-3-(1,3-dioxolan-2-yl)-3-methyl-1-(1,2,4-triazol-1-yl)-butan-2-ol (vgl. DE-OS 3 242 236 sowie DE-OS 3 242 252) der Formel (III)

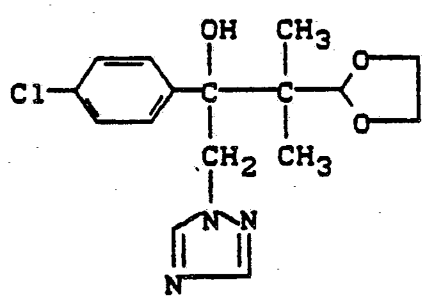

(III)

mit einer Säure, wie beispielsweise wässriger Salzsäure, in Gegenwart eines Verdünnungsmittels wie beispielsweise Ethanol bei Temperaturen zwischen 0 °C und 40 °C umsetzt (s. auch DE-OS 3 334 779 und DE-OS 3 436 452) und den so erhältlichen 3-(4-Chlorphenyl)-2,2-dimethyl-3-hydroxy-4-(1,2,4-triazol-1-yl)-butyraldehyd der Formel (IV)

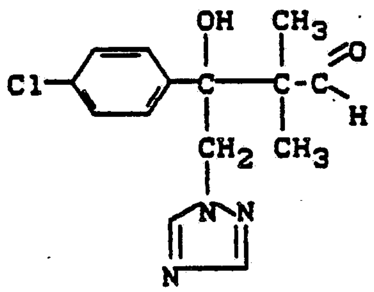

(IV)

mit Hydroxylaminhydrochlorid in Gegenwart eines Verdünnungsmittels wie beispielsweise Ethanol bei Temperaturen zwischen 20 °C und 100 °C umsetzt.

Das erfindungsgemäße Verfahren wird in Gegenwart eines geeigneten wasserabspaltenden Mittels durchgeführt. Als solche kommen alle üblichen Dehydratisierungsmittel infrage (vergl. hierzu beispielsweise C.Ferri "Reaktionen der organischen Synthese", S.572; Thieme Verlag, Stuttgart 1978). Mit besonderem Vorzug verwendet man Carbonsäureanhydride, wie beispielsweise Acetanhydrid.

Als Verdünnungsmittel zur Durchführung des erfindungsgemäßen Verfahrens kommen inerte organische Lösungsmittel infrage, Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Ester, wie Essigsäureethylester.

Es ist auch möglich, bei Verwendung flüssiger, wasserabspaltender Mittel, diese in einem entsprechenden Überschuß gleichzeitig als Verdünnungsmittel einzusetzen.

Das erfindungsgemäße Verfahren wird gegebenenfalls in Gegenwart eines geeigneten Reaktionshilfsmittels durch geführt. Als solche kommen anorganische oder organische Basen in Frage. Hierzu gehören beispielsweise Alkalimetallcarbonate oder -acetate, wie Natriumcarbonat, Natriumacetat, Kaliumcarbonat oder Natriumhydrogencarbonat, sowie tertiäre Amine, wie Triethylamin, N,N-Dimethylanilin, Pyridin oder N,N-Dimethylaminopyridin.

Es ist jedoch auch möglich, das erfindungsgemäße Verfahren ohne Zusatz eines Reaktionshilfsmittels durchzuführen.

Die Reaktionstemperaturen können bei der Durchführung des erfindungsgemäßen Verfahrens in einem größeren Bereich variiert werden. Im allgemeinen arbeitet man bei Temperaturen zwischen 0° C und 120° C, vorzugsweise bei Temperaturen zwischen 40° C und 110° C.

Zur Durchführung des erfindungsgemäßen Verfahrens setzt man pro Mol an 3-(4-Chlorphenyl)-2,2-dimethyl-3-hydroxy-4-(1,2,4-triazol-1-yl)-butyraldehydoxim der Formel (II) im allgemeinen 1.0 bis 50.0 Mol, vorzugsweise 1.0 bis 10.0 Mol an wasserabspaltendem Mittel und gegebenenfalls 1.0 bis 5.0 Mol, vorzugsweise 1.0 bis 2.5 Mol an Reaktionshilfsmittel ein. Die Reaktionsdurchführung, Aufarbeitung und Isolierung des Reaktionsproduktes erfolgt nach allgemein üblichen Methoden.

Zur Herstellung von physiologisch verträglichen Säureadditionssalzen der Verbindung der Formel (I) kommen vorzugsweise folgende Säuren infrage: Halogenwasser stoffsäuren, wie z.B. Chlorwasserstoffsäure

und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Die Säureadditionssalze der Verbindung der Formel (I) können in einfacher Weise nach üblichen Salzbildungsmethoden, wie z.B. durch Lösen der Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäße Verbindung der Formel (I) und readditions-Salze weisen antimikrobielle, insbesondere starke antimykotische Wirkungen auf. Sie besitzt ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tablet ten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder ARt zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, cremes und Gele können neben dem oder den Workstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Workstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethy lensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise von 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise von 5 bis 150 mg/kg Körpergewicht je 24 Stunden und bei parenteraler Applikation in Gesamtmengen von etwa 2,5 bis etwa 50, vorzugsweise von 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführ te Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiel

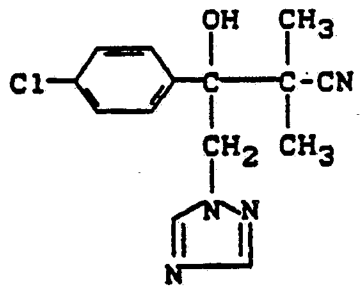

46,3 g (0,15 Mol) 3-(4-Chlorphenyl)-2,2-dimethyl-3-hydroxy-4-(1,2,4-triazol-1-yl)-butyraldehydoxim (Herstellung analog DE-OS 3 334 779) in 500 ml Acetanhydrid werden 3 Stunden auf Rückflußtemperatur erhitzt, dann im Vakuum eingeengt, der Rückstand in 300 ml Dichlormethan aufgenommen, dreimal mit jeweils 200 ml Wasser gewaschen, über Natriumsulfat getrocknet und im Vakuum eingeengt. Der Rückstand kristallisiert beim Verrühren mit Diethylether.

Man erhält 26.1 g (60 % der Theorie) an 3-(4-Chlorphenyl)-2,2-dimethyl-3-hydroxy-4-(1,2,4-triazol-1-yl)-butyronitril vom Schmelzpunkt 123 °C - 124 °C.

Anwendungsbeispiele

In den folgenden Anwendungsbeispielen wurde die nachstehend aufgeführte Verbindung als Vergleichsubstanz eingesetzt:

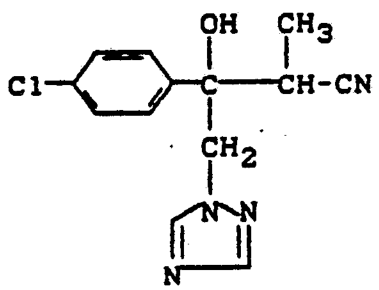

(A)

3-(4-Chlorphenyl)-3-hydroxy-2-methyl-1-(1,2,4-triazol-1-yl)-butyronitril

(bekannt aus EP 106 515)

Beispiel A

Antimykotische in-vitro-Wirksamkeit

Versuchsbeschreibung:

Die in-vitro-Prüfungen wurden mit Keiminokula von durchschnittlich 5 x $10^3$ Keimen/ml Substrat durchgeführt. Als Nährmedium diente Kimmig-Medium.

Die Bebrütungstemperatur betrug 28 °C, die Bebrütungsdauer lag bei 24 bis 96 Stunden bei Hefen und 96 Stunden bei Dermatophyten und Schimmelpilzen.

In diesem Test zeigt die erfindungsgemäße Verbindung 3-(4-Chlorphenyl)-2,2-dimethyl-3-hydroxy-4-(1,2,4-triazol1-yl)-butyronitril der Formel (I) eine deutlich bessere antimykotische Wirksamkeit als die aus dem Stand der Technik bekannte Vergleichsverbindung (A).

**Tabelle A**

**Antimykotische in-vitro-Wirksamkeit**
**MHK*)-Werte in µg/ml Nährmedium**

| Wirkstoff | Trichophyton mentagr. | Microsporum canis | Candida albicans und sp. | Torulopsis glabrata | Aspergillus fumigatus |
|---|---|---|---|---|---|
| (A) (bekannt) 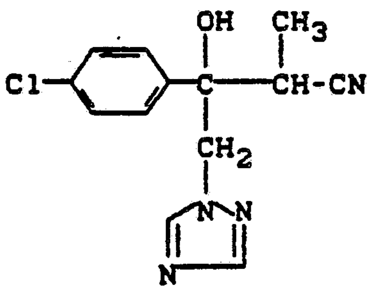 | 32 | 32 | 16-32 | 64 | 64 |
| (I) 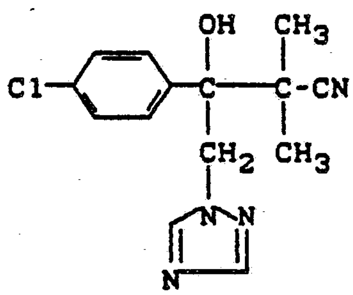 | 4-8 | 4-8 | 4-8 | 32-64 | 64 |

*) minimale Hemmkonzentration

Beispiel B

Antimykotische in-vivo-Wirksamkeit (oral) bei Mäusecandidiose

Versuchsbeschreibung :

Mäuse vom Typ SPF-$CF_1$ werden intravenös mit 1 x $10^6$ logarithmisch wachsenden Candida-Zellen, die in physiologischer Kochsalzlösung suspendiert wurden, infiziert. Gleichzeitig mit der Infektion werden die Tiere mit jeweils 5,0 mg/kg Körpergewicht der Präparate oral behandelt.

Die Behandlung wird 5 Tage fortgesetzt; die Tagesdosis beträgt jeweils 2 mal 5,0 mg/kg Körpergewicht.

Unbehandelte Tiere sterben 3 bis 6 Tage post infektionem. Die Überlebensrate am 6.Tag post infektionem beträgt bei unbehandelten Kontrolltieren etwa 5 %.

In diesem Test zeigt die erfindungsgemäße Verbindung der Formel (I) eine gute Wirkung, während die aus dem Stand der Technik bekannte Verbindung (A) keine Wirkung zeigt.

**Tabelle B**

**Antimykotische in-vivo-Wirkung (oral) bei Mäuse-Candidose**

| Wirkstoff | Wirkung |
|---|---|
| 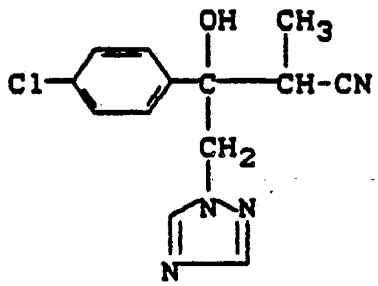 (A) (bekannt) | k.w. |
| 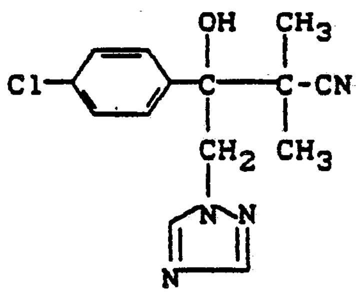 (I) | ++++ |

**Zeichenerklärung:**

| | | |
|---|---|---|
| +++++ | = sehr gute Wirkung | = 90 % Überlebende am 6.Tag.p.i. |
| ++++ | = gute Wirkung | = 80 % Überlebende am 6.Tag.p.i. |
| +++ | = Wirkung | = 60 % Überlebende am 6.Tag.p.i. |
| ++ | = schwache Wirkung | = 40 % Überlebende am 6.Tag.p.i. |
| + | = Spur Wirkung | = unter 40 % Überlebende am 6. Tag p.i. |
| k.W. | | = kein Unterschied zur unbehandelten Infektionskontrolle |

Beispiel C

Antimikrobielle in-vivo-Wirksamkeit (oral) am Modell der experimentellen Meerschweinchen-Trichophytie

Versuchsbeschreibung:

Weiße Meerschweinchen der Rasse Pirbright-white werden auf dem geschorenen, nicht skarifizierten Rücken mit einer Mikro- und Makrokonidien-Suspension von Trichophyton mentagrophytes infiziert.

Die infizierten Tiere werden beginnend mit dem Tag der Infektion 1 x täglich mit 2,5 mg/kg Körpergewicht der Präparate oral behandelt. Die Behandlung wird 10 Tage fortgesetzt; die Tagesdosis beträgt jeweils 1 mal 2,5 mg/kg Körpergewicht.

Bei unbehandelten Tieren entwickelt sich innerhalb von 12 Tagen p.i. das typische Bild einer Dermatophytose mit Rötung, Schuppung und Haarausfall bis zum totalen Integument-Defekt an der Infektionsstelle.

In diesem Test zeigt z.B. die erfindungsgemäße Verbindung der Formel (I) eine gute Wirkung, während die aus dem Stand der Technik bekannte Verbindung A keine Wirkung zeigt.

Tabelle C

Antimikrobielle in-vivo-Wirksamkeit (oral) am Modell der experimentellen Meerschweinchen-Trichophytie

| Wirkstoff | Wirkung |
|---|---|
| 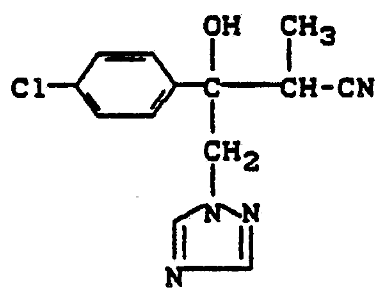 (A) (bekannt) | k.w. |
| 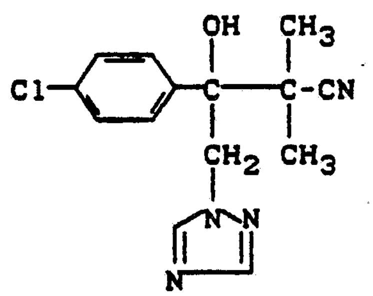 (I) | ++++ |

**Zeichenerklärung:**

| | | | | |
|---|---|---|---|---|
| +++++ | = | sehr gute Wirkung | = | keine Infektionszeichen am 12. bis 15. Tag p.i. |
| ++++ | = | gute Wirkung | = | geringe Rötung, vereinzelt Schuppen |
| +++ | = | Wirkung | = | Rötung, Schuppen ohne Haarausfall |
| ++ | = | schwache Wirkung | = | Rötung, Schuppen, Haarausfall |
| + | = | Spurwirkung | = | flächiger Haarausfall, entzündliche Hautreaktion |
| k.w. | = | keine Wirkung | = | kein Unterschied zur unbehandelten Kontrolle |

Beispiel D / Formulierungen

| 1.) Lösung: | | |
|---|---|---|
| Wirkstoff gemäß Formel (I) | : | 10 g |
| Alkohol, rein (96 %ig) | : | 300 g |
| Isopropylmyristat | : | 526 g |
| | | 836 g |

| 2.) Creme: | | |
|---|---|---|
| Wirkstoff gemäß Formel (I) | : | 10 g |
| Arlacel 60 (Sorbitan-monostearat) | : | 20 g |
| Tween 60 (Polyoxyethylen-(20)-sorbitan-monostearat) | : | 15 g |
| Walrat, künstlich (Mischung von Estern von gesättigten Fettsäuren $C_{14}$-$C_{18}$ und Fettalkoholen $C_{14}$-$C_{18}$) | : | 30 g |
| Lanette O (Gemisch aus Cetyl-Alkohol und Stearylalkohol) | : | 100 g |
| Eutanol G (2-Octyl-dodecanol) | : | 135 g |
| Benzylalkohol | : | 10 g |
| Wasser, entmimeralisiert | : | 680 g |
| | | 1000 g |

## Ansprüche

1. 3-(4-Chlorphenyl)-2,2-dimethyl-3-hydroxy-4-(1,2,4-triazol-1-yl)-butyronitril der Formel (I)

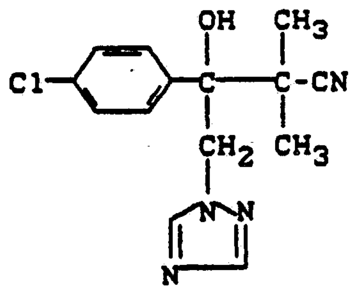

(I)

2. Verfahren zur Herstellung von 3-(4-Chlorphenyl)-2,2-dimethyl-3-hydroxy-4-(1,2,4-triazol-1-yl)-butyronitril aus 2-(4-Chlorphenyl)-3-(1,3-dioxolan-2-yl)-3-methyl-1-(1,2,4-triazol-1-yl)-butan-2-ol (III)

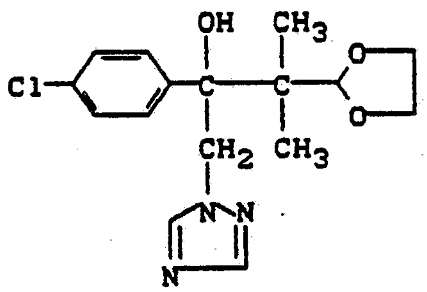

(III)

dadurch gekennzeichnet, daß man die Verbindung (III) mit einer Säure umsetzt, den so erhältlichen 3-(4-Chlorphenyl)-2,2-dimethyl-3-hydroxy-4-(1,2,4-triazol-1-yl)-butyraldehyd (IV)

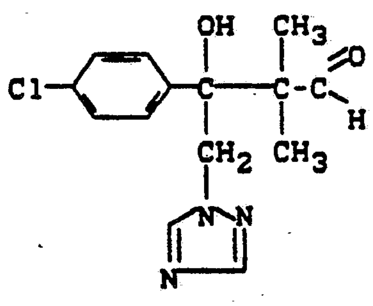

(IV)

mit Hydroxylaminhydrochlorid in Gegenwart eines Verdünnungsmittels umsetzt und das so erhältliche 3-(4-Chlorphenyl)-2,2-dimethyl-2-hydroxy-4-(1,2,4-triazol-1-yl)-butyraldehydoxim (II)

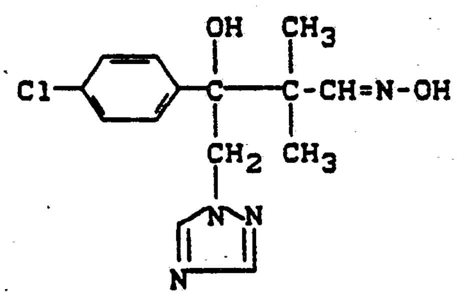

(II)

mit wasserabspaltenden Reagentien zum 3-(4-Chlorphenyl)-2,2-dimethyl-3-hydroxy-4-(1,2,4-triazol-1-yl)-butyronitril (I) dehydratisiert.

3. 3-(4-Chlorphenyl)-2,2-dimethyl-3-hydroxy-4-(1,2,4-triazol-1-yl)-butyronitril sowie dessen physiologisch verträgliche Säureadditionssalze zur Bekämpfung von Krankheiten.

4. 3-(4-Chlorphenyl)-2,2-dimethyl-3-hydroxy-4-(1,2,4-triazol-1-yl)-butyronitril sowie dessen physiologisch verträgliche Säureadditionssalze zur Bekämpfung von Mykosen.

5. Arzneimittel enthaltend 3-(4-Chlorphenyl)-2,2-dimethyl-3-hydroxy-4-(1,2,4-triazol-1-yl)-butyronitril sowie dessen physiologisch verträgliche Säureadditionssalze.

6. Antimykotisches Mittel enthaltend 3-(4-Chlorphenyl)-2,2-dimethyl-3-hydroxy-4-(1,2,4-triazol-1-yl)-butyronitril sowie dessen physiologisch verträgliche Säureadditionssalze.

7. Verwendung von 3-(4-Chlorphenyl)-2,2-dimethyl-3-hydroxy-4-(1,2,4-triazol-1-yl)-butyronitril sowie dessen physiologisch verträgliche Säureadditionssalze bei der Bekämpfung von Krankheiten.

8. Verwendung von 3-(4-Chlorphenyl)-2,2-dimethyl-3-hydroxy-4-(1,2,4-triazol-1-yl)-butyronitril sowie dessen physiologisch verträgliche Säureadditionssalze bei der Bekämpfung von Mykosen.